# EUROPÄISCHE PATENTANMELDUNG

(11) **EP 0 855 196 A1**
(43) Veröffentlichungstag der Anmeldung: **29.07.1998**
(21) Anmeldenummer: 97810041.0
(22) Anmeldetag: 28.01.1997
(51) Int. Cl.: A61N 1/05

(54) **Defibrillationselektrode**

(71) Anmelder: Sulzer Osypka GmbH, 79639 Grenzach-Wyhlen (DE)
(72) Erfinder: Kitschmann, Achim, 79639 Grenzach-Wyhlen (DE)
(74) Vertreter: Heubeck, Bernhard

(57) **Zusammenfassung**

Die Defibrillationselektrode ist für eine temporäre und epikardiale Verwendung nach Herzoperationen bestimmt. Die Elektrode umfasst an ihrem distalen Ende zwecks lokaler Stimulierung der Herzoberfläche einen Draht (1), der distal ein aus einer Verankerungsstelle herausziehbares Ende (11) aufweist. Der Stimulationsdraht oder eine Mehrzahl von Stimulationsdrähten sind derart vorgeformt, dass sie eine globale Einwirkfläche (5) weitgehend gleichmässig sowie mit relativ grossen Krümmungsradien fraktal abdecken. Die Einwirkfläche hat eine weitgehend isodiametrale Form.

## Beschreibung

Die Erfindung betrifft eine Defibrillationselektrode gemäss Oberbegriff von Anspruch 1.

Aus der EP-A 0 636 385 ist ein System zur Defibrillierung des Herzes bekannt. Dieses System ist für eine temporäre Verwendung nach Herzoperationen bestimmt. Für ein mögliches Auftreten von Vorhofflimmern werden zwei Elektroden zu einer gegebenenfalls nötigen Stimulierung der Herztätigkeit implantiert. Stimulationsdrähte, die sich an den distalen Enden der Elektroden befinden, sind epikardial über den Vorhöfen des Herzes angeordnet und am Herzbeutel verankert. Dabei weisen die Drähte distal jeweils Enden auf, die aus Verankerungsstellen herausziehbar sind. Die temporäre Verwendung der Defibrillationselektrode beschränkt sich auf einige Tage nach der Operation; dann ist in der Regel eine Defibrillation nicht mehr nötig, so dass die Elektroden aus dem Körper des Patienten herausgezogen werden können.

Bei dem bekannten System ist der Stimulationsdraht ein gestrecktes oder leicht gebogenes Drahtstück, mit dem ein Streifen der Oberfläche des Vorhofs stimulierbar ist. Für die Defibrillation ist eine relativ grosse Abgabe von Energie notwendig. Um eine Schädigung des Gewebes, das mit dem Stimulationsdraht in Kontakt steht, zu vermeiden, darf die Dichte der abgegebenen Energie nicht zu gross sein.

Es ist Aufgabe der Erfindung, Massnahmen hinsichtlich der bekannten Defibrillationselektrode zu schaffen, aufgrund welcher die Dichte der Stimulationsenergie reduziert werden kann. Diese Aufgabe wird durch die in Anspruch 1 definierte Elektrode gelöst, nämlich dadurch, dass nun neu die Einwirkfläche nicht mehr streifenförmig ist sondern eine weitgehend isodiametrale Form aufweist. Die Einwirkfläche ist ein zusammenhängendes Flächenstück, das in allen Richtungen ungefähr den gleichen Durchmesser D hat. Sie ist von dem vorgeformten Stimulationsdraht oder einer Mehrzahl von vorgeformten Stimulationsdrähten fraktal abgedeckt, wobei unter "fraktal abgedeckt" zu verstehen ist, dass nur ein Teil der Einwirkfläche in direktem Kontakt mit dem Draht steht und dass der Draht über die gesamte Einwirkfläche ausgebreitet ist.

Die erfindungsgemässe Defibrillationselektrode ist für eine temporäre und epikardiale Verwendung nach Herzoperationen bestimmt. Die Elektrode umfasst an ihrem distalen Ende zwecks lokaler Stimulierung der Herzoberfläche einen Draht, der distal ein aus einer Verankerungsstelle herausziehbares Ende aufweist. Der Stimulationsdraht oder eine Mehrzahl von Stimulationsdrähten sind derart vorgeformt, dass sie eine globale Einwirkfläche weitgehend gleichmässig sowie mit relativ grossen Krümmungsradien fraktal abdecken. Die Einwirkfläche hat eine weitgehend isodiametrale Form.

An der Kontaktfläche des Drahtes findet die lokale Stimulierung des Herzes statt. Im Gegensatz zu dieser lokalen Stimulierung erfolgt auf der Einwirkfläche eine globale Einwirkung auf das elektrophysilogische Verhalten des Vorhofs. Diese globale Einwirkfläche soll weitgehend gleichmässig fraktal abdeckt sein; gleichzeitig sollen der Draht bzw. die Drähte relativ grosse Krümmungsradien aufweisen, damit die Elektroden gut aus dem Körper des Patienten entfernbar sind.

Die fraktale Abdeckung durch den Stimulationsdraht oder die Stimulationsdrähte bedeutet im Besonderen folgendes: Ist nur ein Stimulationsdraht vorgesehen, so soll dieser mäandrisch vorgeformt sein, wobei mit Vorteil die minimalen Krümmungsradien des Drahts nicht wesentlich kleiner als ein Zehntel von D sind. Ist eine Mehrzahl von Stimulationsdrähten vorgesehen, so können diese büschelartig oder fächerartig angeordnet sein. Dabei können zumindest einzelne der Stimulationsdrähte nicht oder nur schwach gekrümmt sein; und/oder zumindest einzelne der Drähte können gewellt sein.

Damit der Vorhof des Herzes optimal stimuliert werden kann, muss die Einwirkfläche mindestens rund 1000 mm² gross sein oder - umgerechnet - einen Durchmesser mit einem Wert D grösser als 35 mm haben. Dabei soll die Drahtoberfläche zur lokalen Stimulierung mindestens rund 50 mm² gross sein. Die abgegebenen Energie soll - bezogen auf die Stimulationsfläche - eine Dichte von mindestens rund 0.1 J/mm² haben, damit ein die Defibrillation auslösender Schwellwert überschritten wird. Die Gesamtlänge des Stimulationsdrahts oder der Stimulationsdrähte ist mit Vorteil rund 3 bis 10 Mal grösser als D. Der Durchmesser des Drahts hat mit Vorteil einen Wert im Bereich von 0,1 bis 0,3 mm.

Nachfolgend wird die Erfindung anhand der Zeichnungen erläutert. Es zeigen:
- Fig. 1: eine schematische Darstellung eines Patienten, der mit einem externen Defibrillator verbunden ist,
- Fig. 2: ein Herz mit über den Vorhöfen angeordneten Stimulationsdrähten,
- Fig. 3: eine Hilfsfigur zur Abschätzung von geometrischen Verhältnissen eines mäandrisch vorgeformten Stimulationsdrahts,
- Fig.4-6: Beispiele für die Ausführung der Stimulationsdrähte und
- Fig. 7: eine Kombination eines Herzdrahts mit einer Defibrillationselektrode.

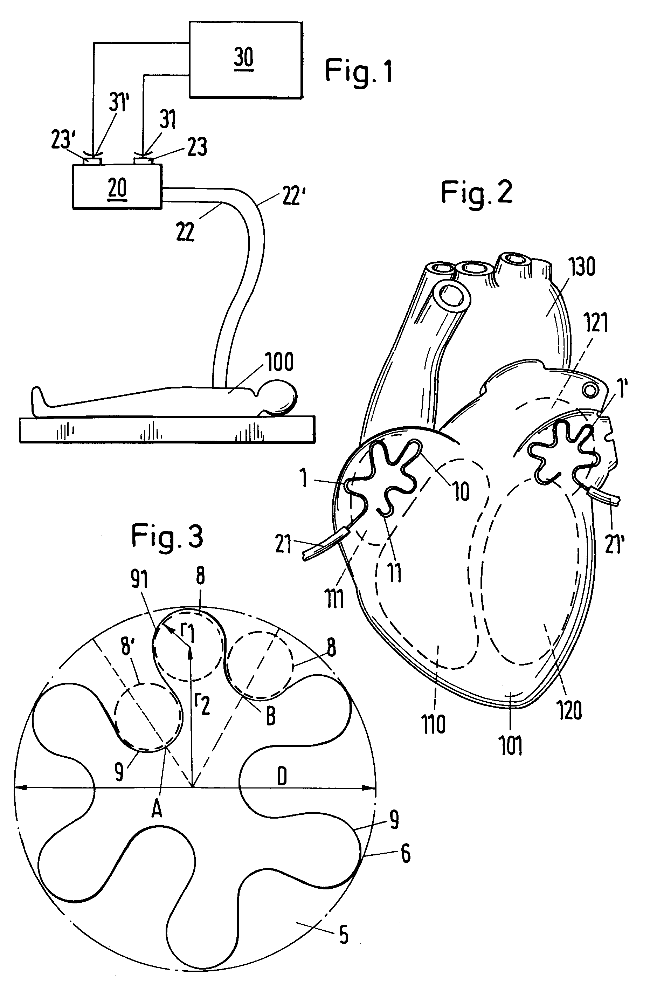

Fig.1 zeigt einen Patienten 100, der über zwei Leitungen 22 sowie 22' und ein Zwischengerät 20 mit einem externen Defibrillator 30 verbunden ist. Der Defibrillator 30 ist ein gebräuchliches Gerät, mit dem eine Defibrillierung durch Auflegen von Oberflächenelektroden 31 und 31' auf die Brust des Patienten durchführbar ist. Die Leitungen 22, 22' stellen die Verbindung zu zwei Defibrillationselektroden 1 und 1' her, die am Herz 101 des Patienten 100 (siehe Fig.2) über den Vorhöfen 111 und 121 temporär implantiert sind. Das Zwischengerät 20, nämlich eine "Begrenzerbox", ist derart konstruiert, dass die Stimulationsenergie auf einen Maximalwert von rund 5 Joule begrenzbar ist. Es handelt sich bei diesem Maximalwert um eine Energiemenge, die wesentlich kleiner ist als die, die durch den Defibrillator 30 im Maximum erzeugbar wäre. Die Begrenzerbox 20 hat metallene Kontaktflächen 23 und 23', mit welchen die Oberflächenelektroden 31 und 31' des externen Defibrillators 30 durch Auflegen in Kontakt gebracht werden können.

Statt des genannten Defibrillators 30 kann selbstverständlich auch ein eigens für implantierte Elektroden konstruiertes Gerät vorgesehen werden, bei dem keine zusätzliche Leistungsbegrenzung nötig ist, so dass die Defibrillationselektroden direkt an dieses Gerät angeschlossen werden können.

Bei dem in Fig.2 dargestellten Herz erkennt man neben der Lage der Vorhöfe 111, 121 auch jene der Herzkammern 110 und 120. Es sind diverse Blutgefässe dargestellt, wobei lediglich die Aorta 130 mit einem Bezugszeichen versehen ist. Bei den Defibrillationselektroden sind ausser den Stimulationsdrähten 1, 1' auch die distalen Enden von Kathetern 21, 21' zu sehen. Diese Katheter 21, 21' verbinden die Drähte 1, 1' mit externen, nicht dargestellten Anschlussstellen an den proximalen Enden der Defibrillationselektroden; an diese Anschlussstellen sind die Leitungen 22, 22' anschliessbar.

Der Draht 1 wirkt lokal auf eine Stimulationsfläche 10, die durch den direkten Kontakt zwischen der Drahtoberfläche und der Herzoberfläche gegeben ist. Um die Lage des Drahts sichern zu können, sind Verankerungen vorzusehen. Dazu kann beispielsweise die Drahtspitze 11 über ein kurzes Stück durch das oberflächliche Gewebe des Epikards hindurchgezogen werden. Der Draht 1 kann aber auch angenäht werden (vgl. dazu die genannte EP-A 0 636 385).

Fig.3 zeigt eine geschlossene, symmetrische Kurve 9, die eine kreisförmige Fläche 5 (Durchmesser D, Peripherie 6) fraktal abdeckt. Für diese Kurve 9 lässt sich einfach bestimmen, welches Verhältnis zwischen dem Umfang U der Kurve 9 und D besteht: Die Kurve 9 umschlingt zwölf an der Peripherie 6 angeordnete kleine Kreise 8 (Radius r₁) und sechs gleich grosse Kreise 8', die sich im mittleren Bereich der Fläche 5 befinden. Das Teilstück 91 der Kurve 9 zwischen den Punkten A und B wiederholt sich 6 Mal. Jedes Teilstück 91 ist ungefähr 5/4 Mal den Umfang des kleinen Kreises 8 lang. Der Durchmesser D hat ungefähr 10 Mal die Länge des Radius r₁. Somit ergibt sich: U = 1.5 π D. Der Umfang der Kurve 9 ist somit rund 5 Mal grösser als der Durchmesser D.

Für einen mäandrisch geformten Stimulationsdraht 1 bestehen grössenordnungsmässig die gleichen Verhältnisse wie für die Kurve 9 der Fig.3. Auch für Elektroden mit einer Mehrzahl von Stimulationsdrähten kann das gleiche Verhältnis zwischen Gesamtdrahtlänge und Durchmesser der Einwirkfläche vorgesehen werden. Dieses Verhältnis liegt im Bereich zwischen rund 3 und 10. Für den minimalen Krümmungsradius gilt, dass er nicht wesentlich kleiner als der Radius r₁ der Kreise 8 (≈ 0.1 D) sein soll.

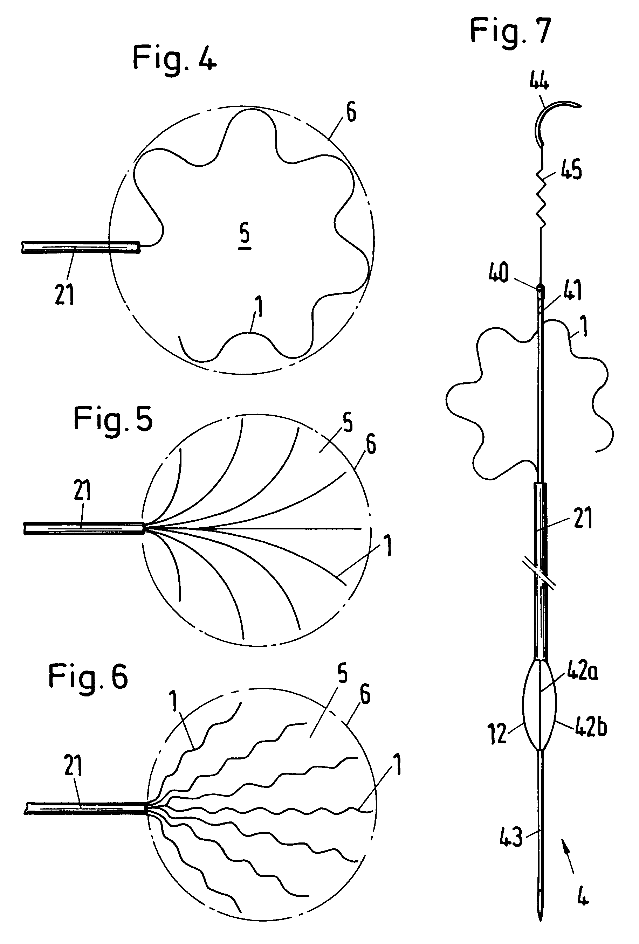

Fig.4 zeigt ein Ausführungsbeispiel mit einem mäandrisch vorgeformten Stimulationsdraht, dessen fraktale Abdeckung der Einwirkfläche 5 etwas weniger gleichmässig ist als bei der Kurve 9 der Fig.3. Die Figuren 5 und 6 zeigen Elektroden mit büschelartig oder fächerartig angeordneten Stimulationsdrähten, im einen Beispiel mit nicht oder nur schwach gekrümmten Drähten und im anderen Beispiel mit gewellten Drähten.

Die in Fig.7 gezeigte Elektrode 4 stellt einen Herzdraht dar, der mit einer Defibrillationselektrode (Stimulationsdraht 1, Katheter 21) kombiniert ist. Der Herzdraht dient zur zeitweiligen Überwachung und Stimulierung der Herztätigkeit ("Pacen") nach einer Herzoperation. Er ist an einen äusseren Herzschrittmacher und/oder einen EKG-Monitor anschliessbar.

Die Kombination von Defibrillationselektrode und Herzdraht weist zusätzlich zu dem Stimulationsdraht bzw. den Stimulationsdrähten Elektrodenteile für EKG-Messungen und zum Pacen auf. Der Herzdraht umfasst folgende Teile: einen Katheter 41 mit elektrischen Verbindungen; zwei unisolierte Drahtstücke 42a und 42b, die als äussere Anschlussstellen der elektrischen Verbindung vorgesehen sind; eine Elektrode 40 in Form einer Hülse aus Platin, mit der ein elektrischer Kontakt zum Herzmuskel herstellbar ist; und ein zick-zack-förmiger Kunststofffaden 45, mit dem der Herzdraht 4 im Herzmuskel verankerbar ist. An den beiden Enden des Herzdrahts 4 befinden sich eine Brustnadel 43 bzw. eine Herznadel 44, die Operationshilfen sind und die nach der zweckgemässen Verwendung jeweils abgeschnitten werden. Ein äusserer Anschlussdraht 12 des Stimulationsdrahts 1 wird an einen HV-sicheren Stecker angeschlossen.

Ähnlich wie beim Herzdraht der zick-zack-förmige Kunststofffaden 45 können entsprechende Mittel zur Verankerung bei der Defibrillationselektrode an den oder an einzelnen distalen Enden der Stimulationsdrähte vorgesehen sein.

Die Leitung, die das proximale Ende der Defibrillationselektrode mit dem Stimulationsdraht bzw. den Stimulationsdrähten verbindet, muss elektrisch gut leitend sein. Mit Vorteil weist sie einen Kern aus Silber und einen Mantel aus Edelstahl auf.

Die Kombination von Defibrillationselektrode und Herzdraht ist eine vorteilhafte Ausführungsform der Erfindung. Gegenstand der Erfindung ist aber auch eine Defibrillationselektrode ohne zusätzliche Komponenten eines Herzdrahts.

## Patentansprüche

1. Defibrillationselektrode, die für eine temporäre und epikardiale Verwendung nach Herzoperationen bestimmt ist, welche Elektrode an ihrem distalen Ende zwecks lokaler Stimulierung der Herzoberfläche einen Draht (1) umfasst, der distal ein aus einer Verankerungsstelle herausziehbares Ende (11) aufweist,
dadurch gekennzeichnet, dass der Stimulationsdraht oder eine Mehrzahl von Stimulationsdrähten derart vorgeformt sind, dass sie eine globale Einwirkfläche (5) weitgehend gleichmässig sowie mit relativ grossen Krümmungsradien fraktal abdecken und dass die Einwirkfläche eine weitgehend isodiametrale Form hat.

2. Defibrillationselektrode nach Anspruch 1, dadurch gekennzeichnet, dass die Einwirkfläche (5) mindestens rund 1000 mm² gross ist.

3. Defibrillationselektrode nach Anspruch 1 oder 2, dadurch gekennzeichnet, dass die Drahtoberfläche (10) zur lokalen Stimulierung mindestens rund 50 mm² gross ist und dass eine Dichte der abgegebenen Energie von mindestens rund 0.1 J/mm² vorgesehen ist, wobei diese Dichte auf die Stimulationsfläche (10) bezogen ist.

4. Defibrillationselektrode nach Anspruch 1 oder 2, dadurch gekennzeichnet, dass die Gesamtlänge des Stimulationsdrahts (1) oder der Stimulationsdrähte rund 3 bis 10 Mal grösser als der mittlere Durchmesser D der Einwirkfläche (5) ist und dass der Drahtdurchmesser einen Wert im Bereich von 0,1 bis 0,3 mm hat.

5. Defibrillationselektrode nach einem der Ansprüche 1 bis 4, dadurch gekennzeichnet, dass nur ein Stimulationsdraht (1) vorgesehen ist, der mäandrisch vorgeformt ist, wobei insbesondere die minimalen Krümmungsradien des Drahts nicht wesentlich kleiner als ein Zehntel des mittleren Durchmessers D der Einwirkfläche (5) sind.

6. Defibrillationselektrode nach einem der Ansprüche 1 bis 5, dadurch gekennzeichnet, dass eine Mehrzahl von Stimulationsdrähten (1) vorgesehen ist, die büschelartig oder fächerartig angeordnet sind.

7. Defibrillationselektrode nach Anspruch 6, dadurch gekennzeichnet, dass zumindest einzelne der Stimulationsdrähte (1) nicht oder nur schwach gekrümmt sind und/oder dass zumindest einzelne der Drähte gewellt sind.

8. Defibrillationselektrode nach einem der Ansprüche 1 bis 7, dadurch gekennzeichnet, dass mindestens einzelne der Stimulationsdrähte (1) an ihren Enden (11) jeweils ein Verankerungsmittel aufweisen.

9. Defibrillationselektrode nach einem der Ansprüche 1 bis 8, dadurch gekennzeichnet, dass zwischen einem proximalen Ende und dem Stimulationsdraht (1) bzw. den Stimulationsdrähten Verbindungsleitungen angeordnet sind, die jeweils einen Kern aus Silber und einen Mantel aus Edelstahl aufweisen.

10. Defibrillationselektrode nach einem der Ansprüche 1 bis 9, dadurch gekennzeichnet, dass zusätzlich zu dem Stimulationsdraht (1) bzw. den Stimulationsdrähten Elektrodenteile (40, 41) für EKG-Messungen vorgesehen sind.

11. Vorrichtung mit zwei Defibrillationselektroden gemäss einem der Ansprüche 1 bis 10 und einer Energiequelle für die Stimulation, dadurch gekennzeichnet, dass die Energiequelle ein konventioneller, extrakorporal angeordneter Defibrillator (30) ist und dass ein Gerät (20) zur Begrenzung der Stimulationsenergie - vorzugsweise auf einen Maximalwert von rund 5 Joule - zwischen dem Defibrillator und den Elektroden vorgesehen ist.
